# EUROPÄISCHE PATENTSCHRIFT

(11) **EP 0 990 641 B1**
(45) Veröffentlichungstag und Bekanntmachung des Hinweises auf die Patenterteilung: **17.03.2004**
(21) Anmeldenummer: 99116197.7
(22) Anmeldetag: 24.08.1999
(51) Int. Cl.: C07C 231/12, C07C 269/06, C07C 233/06, C07C 271/24

(54) **Verfahren zur Synthese alpha-substituierter Ringsysteme**
Process for the synthesis of alpha-substituted ring systems
Procédé de synthèse de systèmes cycliques substitués en alpha

(30) Priorität: 03.09.1998 DE 19840108
(43) Veröffentlichungstag der Anmeldung: 05.04.2000
(73) Patentinhaber: Bayer Chemicals AG, 51368 Leverkusen (DE)
(72) Erfinder: Gürtler, Christoph, Dr., 50676 Köln (DE); Jautelat, Manfred, Dr., 51399 Burscheid (DE); Greiving, Helmut, Dr., 51375 Leverkusen (DE); Hugl, Herbert, Dr., 51467 Bergisch Gladbach (DE)

(56) Entgegenhaltungen:
- PATENT ABSTRACTS OF JAPAN vol. 014, no. 020 (C-676), 17. Januar 1990 (1990-01-17) & JP 01 261354 A (SUMITOMO CHEM CO LTD), 18. Oktober 1989 (1989-10-18)
- ROSHAN JUMNAH ET AL.: "Synthesis of N-Protected Amino Esters via Palladium Catalysed Allylic Substitution" TETRAHEDRON LETTERS, Bd. 34, Nr. 41, 8. Oktober 1993 (1993-10-08), Seiten 6619-6622, XP002124526 OXFORD GB
- SUSAN K. ARMSTRONG: "Ring Closing Diene Methathesis in Organic Synthesis" JOURNAL OF THE CHEMICAL SOCIETY, PERKIN TRANSACTIONS 1, Nr. 2, 21. Januar 1998 (1998-01-21), Seiten 371-388, XP002124527 LETCHWORTH GB

## Beschreibung

Durch das erfindungsgemäße Verfahren wird mit wenigen Reaktionsschritten ein neuer, vorteilhafter Zugang zu α-substituierten Ringsystemen bereitgestellt.

Zu diesen Ringsystemen zählen auch die Tetrahydroaniline. Diese sind interessante Vorprodukte von Feinchemikalien oder auch Pflanzenschutzmitteln, da an der Doppelbindung weitere Transformationen möglich sind. Sie sind weiterhin eine mögliche Quelle für Cyclohexylamin, das als Vulkanisationsbeschleuniger bzw. Korrosionsinhibitor und als Vorstufe von synthetischen Süßstoffen (z.B. für Cyclamat) Verwendung findet.

Nach dem bisher bekannt gewordenen Stand der Technik sind zur Herstellung von Tetrahydroanilinen eine Vielzahl von Stufen bzw. Teilschritten erforderlich: P. Kocovsky (*Synlett* **1990,** 677) beschreibt einen Zugang zu Tetrahydroanilinderivaten durch Umsetzung von Cyclohexenon mit Hydroxylamin in Pyridin zum Cyclohexenonoxim, das anschließend in Pyridin acyliert und dann mit Lithium-Aluminium-Hydrid reduziert wird. Mit diesem Verfahren werden zwar gute Ausbeuten erzielt, eine Reduktion mit Lithium-Aluminium-Hydrid ist für eine industrielle Anwendung bzw. die Herstellung großer Mengen jedoch nicht geeignet.

Y. Yang, F. Diederich und J. S. Valentine (*J. Am. Chem. Soc*. **1991,** *113*, 7195 und *J*. *Am. Chem*. *Soc*, **1990,** *112*, 7826) beschreiben die Oxidation von Cyclohexen mit Iodosylbenzen in Gegenwart von Aluminiumtriflat und Acetonitril in Ausbeuten von maximal 13 %. T. Shono und A. Ikeda (*J. Am. Chem. Soc.* **1972,** *94*, 7892) legen die Elektrooxidation von Cyclohexen in Acetonitril mit geringen Mengen Wasser dar, erhalten das gewünschte Tetrahydroanilinderivat allerdings nur als Nebenprodukt. Eine Aussage über die Ausbeute wird nicht gegeben. T. Toshimitsu, H. Owada, T. Aoai, S. Uemura und M. Okana (*J. C. S, Chem. Commun*. **1981,** 546) führen eine Wasserstoffperoxidoxidation von α-Selenylphenyl-acetamidocyclohexan durch und gelangen bei Temperaturen von 250°C in sehr guten Ausbeuten zum gewünschten Tetrahydroanilinderivat, allerdings unter Zuhilfenahme des industriell kaum verwendbaren Selens.

R. Jumnah, M. J. Williams, A. C. Williams (*Tetrahedron Letters* **1993,** *34,* 6619) und J. F. Bower, R. Jumnah, A. C. Williams, J. M. J. Williams (*J. Chem. Soc., Perkin Trans. 1* **1997,** 1411) beschreiben ein Verfahren zur Herstellung eines tosylierten Tetrahydroanilinderivats durch Umsetzung von O-Acyl-2-Cyclohexenol mit Tosylamid unter Palladiumkatalyse. Ebenfalls beschrieben wird ein Weg ausgehend von O-Acetyl-2-Cyclohexenol durch palladiumkatalysierte Umsetzung mit einem Azidion zum Azido-2-cyclohexen und anschließende Transformation des Azids mit Thioessigsäure zum Acetamido-2-Cyclohexen mit einer Gesamtausbeute von 46 %. Diese Verfahren weisen eine hohe Stufenzahl auf und es werden z. T. schlecht zu handhabende Edukte (Azid) eingesetzt.

Y. Ichikawa, M. Yamazaki, M. Isobe (*J. Chem. Soc. Perkin Trans.1*, **1993,** 2429) sowie Y. Ichikawa, M. Osuda, I. I. Ohtani, M. Isobe (*J. Chem. Soc., Perkin Trans. 1* **1997**, 1449) schildern ein Verfahren, bei dem zunächst 2-Cyclohexenol mit Trichloracetylisocyanat und Kaliumcarbonat zum O-Carboxyamid-2-cyclohexenol umgesetzt wird, das in einem Folgeschritt zum O-Cyano-2-cyclohexenol dehydratisiert wird, das dann wiederum zum 2-Cyclohexenylisocyanat mit guten Ausbeuten umgelagert wird. Dieses 2-Cyclohexenylisocyanat wird dann mit Trimethylaluminium zum gewünschten Tetrahydroanilinderivat umgelagert. Besonders nachteilig wirkt sich hierbei aus, daß stöchiometrische Mengen Trichloracetylisocyanat und für diese Umlagerung schlecht zu handhabendes Trimethylaluminium benötigt werden.

C. Briguet, C. Freppel, J.-C. Richer und M. Zador (*Can. J. Chem*. **1974,** *52*, 3204) beschreiben ein Verfahren zur Oxidation von Cyclohexen mit Cerammoniumnitrat in Acetonitril, das 1 % Wasser enthält und erhalten das Acetamido-2-cyclohexen. Hier ist die stöchiometrische Verwendung von Cerammoniumnitrat für eine industrielle Anwendung hinderlich. Y. Leblanc, R. Zamboni, M. A. Bernstein (*J. Org. Chem*. **1991,** *56*, 1971) schildern eine En-Reaktion von Cyclohexen mit Bis-2,2,2-trichlorethylazodicarboxylat bei 155°C, das nach Aufarbeitung eine Ausbeute von 70 % an gewünschtem Tetrahydroanilinderivat liefert.

G. Kresze und H. Münsterer (*J. Org. Chem*. **1983,** *48* 3561) beschreiben eine En-Reaktion von Cyclohexen mit Bis(methoxycarbonyl)schwefeldiimid zu einem Produkt, das nach basischer Aufarbeitung ein Tetrahydroanilinderivat liefert. In den beiden letzten Fällen steht das in stöchiometrischen Mengen zu verwendende Reagenz einer kommerziellen Nutzung im Wege.

In JP-A-01 261 354 (Sumitomo Corp.) ist das beste bislang bekannt gewordene industrielle Verfahren zur Synthese von Tetrahydroanilinderivaten beschrieben worden. 1,2-Dichlorhexan wird im Autoklaven in Gegenwart von Ammoniak in Isopropanol in einer Ausbeute von 80 % zum 2-Cyclohexenamin umgesetzt. Der Umstand, daß zunächst 1,2-Dichlorhexan gewonnen werden muß und der hohe apparative Aufwand zur Durchführung von Autoklavenreaktionen lassen dieses Verfahren aber als nicht besonders vorteilhaft erscheinen.

Es bestand daher der Bedarf nach einem einfacheren Zugang zum Tetrahydroanilin bei deutlicher Entlastung der Umwelt und gleichzeitiger Reduktion der Produktionskosten.

Diese Aufgabe wird erfindungsgemäß durch eine Ringschlußmetathesereaktion, die ausgehend von terminalen 3-Amino-octadienen zu den entsprechenden ringgeschlossenen Verbindungen führt, gelöst. Eine derartige Ringschlußmetathesereaktion ist bisher nicht bekannt geworden.

Der vorliegenden Erfindung lag auch die Aufgabe zugrunde, ein universell anwendbares Verfahren bereitzustellen, mit dem außer Tetrahydroanilin auch andere, gegebenenfalls größere, α-funktionalisierte ungesättigte Ringsysteme der Formel (II) erhältlich sind.

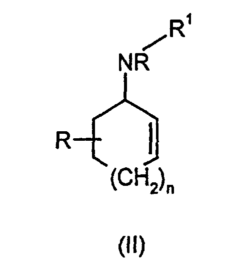

Bei der Olefinmetathese funktionalisierter terminaler Diene in Gegenwart von Edelmetallkatalysatoren wird ein Ringschluß des Diens induziert, wobei bei dieser Reaktion Ethylen als weiteres Wertprodukt gewonnen wird (einen Überblick über diesen Reaktionstyp geben z.B. M. Schuster, S. Blechert, *Angew. Chem*. **1997,** *109,* 2124 und S. Armstrong, *J. Chem. Soc*., *Perkin Trans. 1,* **1998,** 371). Die als Ausgangsprodukte benötigten Diene können leicht durch eine sogenannte Telomerisierungsreaktion oder auf andere, an sich bekannten Reaktionswegen gewonnen werden.

Die Telomerisationsreaktion (beschrieben z.B. von T. Prinz, W. Keim, B. Drießen-Hölscher, *Angew. Chem.* **1996,** *108*, 1835) kommt mit sehr einfachen und leicht zugänglichen Edukten aus, nämlich Ammoniak und Butadien.

Erfindungsgemäß wird dieses Edukt bevorzugt in einer geschützten Form eingesetzt, besonders bevorzugt in seiner acylierten Form. Im allgemeinen wird man das Formamid einsetzen, es sind aber auch andere Schutzgruppen wie z.B. Carbamate anwendbar. Die Schutzgruppen lassen sich leicht entfernen bzw. in einer Gleichgewichtsreaktion auf das Edukt übertragen, so daß dieser Hilfsstoff vollständig recyclierbar ist.

Als Katalysatoren werden im erfindungsgemäßen Verfahren die in WO-A-93/20111 beschriebenen Ruthenium-Alkylidenverbindungen, die von A. W. Stumpf, E. Saive, A. Deomceau und A. F. Noels in *J. Chem. Soc*., *Chem. Commun*. **1995,** 1127-1128 beschriebenen Katalysatorsysteme auf Rutheniumbasis oder die von P. Schwab, R. H. Grubbs und J. W. Ziller in *J. Am. Chem. Soc*. **1996,** *118*, 100 veröffentlichten Katalysatorsysteme bevorzugt verwandt.

Besonders bevorzugt wird das Bis(tricyclohexylphosphin)benzylidenruthenium(IV)-dichlorid eingesetzt.

Durch das erfindungsgemäße Verfahren wird aus einem α-funktionalisierten Dien, bevorzugt einem 1,7-Octadien aber auch einem längerkettigen Dien quantitativ ein cyclisches ungesättigtes Ringsystem der Formel (II) erhalten.

In einer bevorzugten Ausführungsform der Erfindung wird permanent unter Schutzgasatmosphäre gearbeitet, so daß der Katalysator über mehrere Zyklen eingesetzt werden kann.

Die Katalysatormenge beim erfindungsgemäßen Verfahren liegt in der Regel bei 0.001 bis 10 mol-%, bezogen auf die Verbindung der Formel (I), bevorzugt wird man die Reaktion bei 0.1 bis 1 mol-% durchführen.

Die Reaktionszeit ist variabel und hängt von der Reaktionstemperatur, dem Reaktionsdruck und der Art und der Menge des Katalysators ab. Normalerweise beträgt die Reaktionszeit 0.01 bis 30 Stunden, bevorzugt 1 bis 10 Stunden.

Der im erfindungsgemäßen Verfahren anzuwendende Druck ist kein kritischer Parameter. Es können auch Unterdrücke bis 0.01 bar und Überdrücke bis beispielsweise 100 bar verwandt werden, bevorzugt jedoch 0.1 bis 10 bar absoluter Druck, besonders bevorzugt Atmosphärendruck.

Das erfindungsgemäße Verfahren erlaubt die Ringschlußmetathese in einem Lösungsmittel oder ohne die Verwendung eines Lösungsmittels. Das Lösungsmittel kann wirksam zur Steuerung der Reaktionsgeschwindigkeit eingesetzt werden. Das geeignete Lösungsmittel für ein ausgewähltes Substrat kann durch den Fachmann im vorliegenden Fall in einer üblichen Reihenuntersuchung ohne großen Aufwand ermittelt werden. Das Lösungsmittel ist bevorzugt ein inertes organisches Lösungsmittel, zum Beispiel unter Reaktionsbedingungen flüssige aliphatische Kohlenwasserstoffe mit 4 bis 20 Kohlenstoffatomen wie Butan, Pentan, Hexan oder Heptan, Chlor- und Bromkohlenwasserstoffe, zum Beispiel Dichlormethan, Chloroform, Bromoform, Iodoform oder auch chlorierte oder bromierte Benzole, oder aromatische Kohlenwasserstoffe wie Benzol, Toluol oder Xylol, wobei diese nur beispielhaft zu verstehen sind. Daneben ist auch die Nutzung von Ethern (z.B. Diphenylether) möglich. Bevorzugt ist die Verwendung von Benzol oder Toluol.

Die Reaktionstemperatur ist kein kritischer Parameter. Im allgemeinen wird die Reaktionstemperatur durch den Siedepunkt des verwandten Lösungsmittels bzw. durch die Siedepunkte der Edukte bestimmt, sofern nicht durch die Einwirkung von Druck diesem entgegengewirkt wird.

### Allgemeine Reaktionsgleichung des Verfahrens

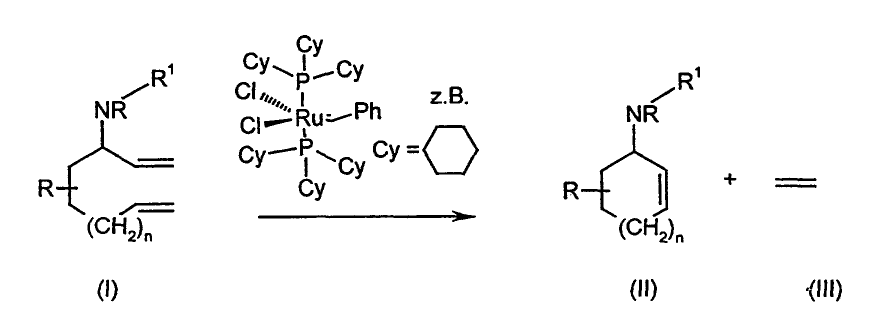

Hierin bedeuten jeweils unabhängig voneinander
- R: einen oder mehrere weitere organische Substituenten, bevorzugt Wasserstoff, gegebenenfalls anelliertes Aryl, Alkyl, -CN, -COOR¹, Halogen,
- R¹: -COR, -SO₂PhR, -COOR, CONRR¹, CONRR, *tert*.-Butyl, PR₂ oder PR²₂
- R²: Alkyl, Phenyl
- R und R¹: gemeinsam 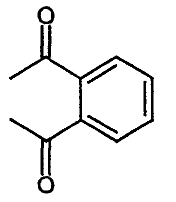
und
- n: die Zahlen 1,2,3 oder 4, bevorzugt 1 oder 2, besonders bevorzugt 1.

Die Doppelbindung des resultierenden Moleküls (II) kann ebenfalls durch mindestens einen Rest R substituiert sein.

Die Art des Substituenten R ist nicht wesentlich für die Erfindung, es können im Prinzip alle in der organischen Chemie gängigen Reste verwendet werden. Die bevorzugten Alkylgruppen R sind erfindungsgemäß lineare oder verzweigte C₁- bis C₈-Alkylgruppen, besonders bevorzugt lineare C₁- bis C₄-Alkylgruppen, bei den Arylgruppen handelt es sich bevorzugt um Phenylgruppen.

### Beispiele

### Beispiel 1: N-Acetyl-3,4,5,6-tetrahydroanilin

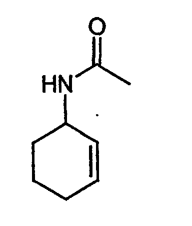

176 mg (1 mmol) N-Acetyl-3-Amino-1,7-octadien und 7 mg Bis(tricyclohexylphosphin)benzylidenruthenium(IV)dichlorid (ca 1 mol-%) werden in einem ausgeheizten Schlenk-Rohr unter Argon-Atmosphäre in 2 ml abs. Benzol gelöst. Man läßt ca. 14 h bei 80°C reagieren. Zur Aufarbeitung filtriert man über eine sehr kurze Kieselgel-Filtriersäule (0,5 cm) (Laufmittel: Ether) und engt ein.
Ausbeute: 138 mg N-Acetyl-3,4,5,6-tetrahydroanilin (0.99 mmol, 99 %).
R_{*f*}-Wert (Laufmittel: Petrolether:Essigester 2:1, stationäre Phase (DC): SiO₂): 0.22; ¹H-NMR (400 MHz, CDCl₃) δ = 5.85 (1H, d, *J*= 9.0 Hz), 5.60 (1H, m), 5.58 (1H, d, *J* = 9.0 Hz), 4.48 (1H, m), 2.00 (2H, m), 1.95 (3H, s), 1.91 (1H, dddd, *J* = 12.0, 11.0, 6.0, 6.0 Hz), 1.65 (2H, tt, *J =* 6.0, 6.0 Hz), 1.55 (1H, dddd, *J* = 12.0, 11.0, 6.0, 6.0 Hz).

### Beispiel 2: N-Carboxymethyl-3,4,5,6-tetrahydroanilin

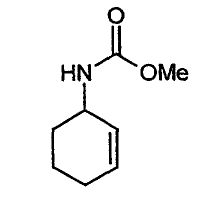

92 mg (0.5 mmol) N-Carboxymethyl-3-Amino-1,7-octadien und 4 mg Bis(tricyclohexylphosphin)benzylidcnruthenium(IV)dichlorid (ca 1 mol-%) werden in einem ausgeheizten Schlenk-Rohr unter Argon-Atmosphäre in 1 ml abs. Benzol gelöst. Man läßt ca. 14 h bei 80°C reagieren. Diese Reaktion verläuft mit gleichem Ergebnis und in gleicher Zeitdauer auch in Chloroform (Kp. 56°C). Zur Aufarbeitung filtriert man über eine sehr kurze Kieselgel-Filtriersäule (0,5 cm) und wäscht vier mal mit je 1 ml Acetonitril nach und engt ein.
Ausbeute: 77 mg N-Carboxymethyl-3,4,5,6-tetrahydroanilin (0.49 mmol, 99 %).
¹H NMR (400 MHz, CDCl₃) δ 5.85 (1H, d, *J*= 9.0 Hz), 5.60 (1H, d, *J*= 9.0 Hz), 4.70 (1H, s), 4.20 (1H, s), 3.65 (3H, s), 1.98 (2H, m), 1.90 (1H, m), 1.62 (2H, m), 1.52 (1H, m).

## Patentansprüche

1. Verfahren zur Herstellung von zumindest α-substituierten Ringsystemen der Formel (II) 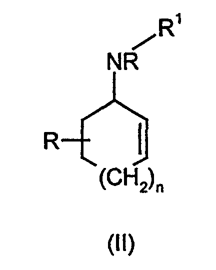 in welcher jeweils unabhängig voneinander
R einen oder mehrere organische Substituenten, bevorzugt Wasserstoff, gegebenenfalls anelliertes Aryl, Alkyl, -CN, -COOR¹, Halogen,
R¹ -COR, -SO₂PhR, -COOR, CONRR¹, CONRR, *tert*.-Butyl, PR₂ oder PR²₂ und
R² Alkyl, Phenyl
und
R und R¹ gemeinsam 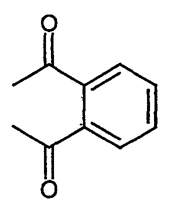
bedeuten
und
n die Zahlen 1,2,3 oder 4, bevorzugt 1 oder 2, besonders bevorzugt 1,
bedeuten
und auch die Doppelbindung durch mindestens einen Rest R substituiert sein kann,
in welchem eine Verbindung der Formel (I) 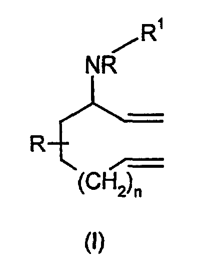 in welcher
R, R¹ und n die oben angegebene Bedeutung haben,
in Gegenwart eines Edelmetallkatalysators einer Metathesereaktion unterworfen wird.

2. Verfahren gemäß Anspruch 1, **dadurch gekennzeichnet, daß** als Edelmetallkatalysator ein Rutheniumkomplex eingesetzt wird.

3. Verfahren gemäß Ansprüchen 1 und 2, **dadurch gekennzeichnet, daß** es sich bei dem Edelmetallkomplex um Bis(tricyclohexylphosphin)benzylidenruthenium(IV)dichlorid handelt.

4. Verfahren gemäß Ansprüchen 1 bis 3, **dadurch gekennzeichnet, daß** R in Formeln (I) und (II) die Bedeutung Wasserstoff, gegebenenfalls anelliertes Aryl, Alkyl, -CN, -COOR¹ oder Halogen hat.

5. Verfahren gemäß Ansprüchen 1 bis 4, **dadurch gekennzeichnet, daß** n in Formeln (I) und (II) die Bedeutung 1 oder 2 hat.

6. Verfahren gemäß Ansprüchen 1 bis 4, **dadurch gekennzeichnet, daß** n in Formeln (I) und (II) die Bedeutung 1 hat.

7. Verfahren gemäß Ansprüchen 1 bis 6, **dadurch gekennzeichnet, daß** in Formeln (I) und (II) R¹ und R Wasserstoff bedeuten und n den Wert 1 hat.

## Claims

1. Process for preparing at least α-substituted ring systems of the formula (II) 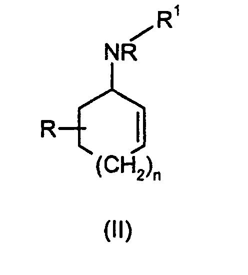 in which, in each case independently of one another,
R represents one or more organic substituents, preferably hydrogen, optionally fused aryl, alkyl, -CN, -COOR¹, halogen,
R¹ represents -COR, -SO₂PhR, -COOR, CONRR¹, CONRR, *tert*-butyl, PR₂ or PR²₂ and
R² represents alkyl, phenyl
and
R and R¹ together represent 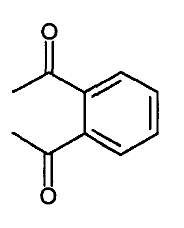
and
n represents the numbers 1, 2, 3 or 4, preferably 1 or 2, particularly preferably 1,
and even the double bond may be substituted by at least one radical R,
which comprises subjecting a compound of the formula (I) 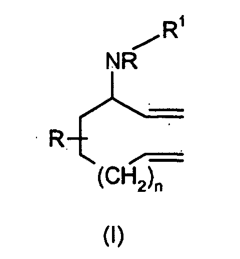 in which
R, R¹ and n are each as defined above,
to a metathesis reaction in the presence of a noble metal catalyst.

2. Process according to Claim 1, **characterized in that** the noble metal catalyst used is a ruthenium complex.

3. Process according to Claims 1 and 2, **characterized in that** the noble metal complex is bis(tricyclohexylphosphine)benzylideneruthenium(IV) dichloride.

4. Process according to Claims 1 to 3, **characterized in that** R in the formulae (1) and (II) represents hydrogen, optionally fused aryl, alkyl, -CN, -COOR¹ or halogen.

5. Process according to Claims 1 to 4, **characterized in that** n in the formulae (I) and (II) represents 1 or 2.

6. Process according to Claims 1 to 4, **characterized in that** n in the formulae (I) and (II) represents 1.

7. Process according to Claims 1 to 6, **characterized in that** in the formulae (I) and (II) R¹ and R each represent hydrogen and n represents 1.

## Revendications

1. Procédé de synthèse de systèmes cycliques substitués au moins en a de formule II 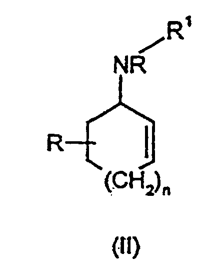 dans laquelle, indépendamment l'un de l'autre,
R désigne un ou plusieurs substituants organiques, de préférence l'hydrogène un radical, éventuellement à noyaux condensés, aryle, alkyle, -CN, -COOR¹, un halogène,
R¹ désigne -COR, -SO₂PhR, -COOR, CONRR¹, CONRR, *tert*.-butyle, PR² ou PR²₂ et
R² un radical alkyle ou phényle
et
R et R¹ désignent ensemble 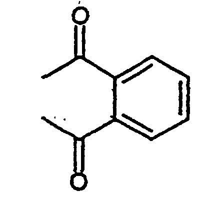
et
n désigne les nombres 1, 2, 3 ou 4, de préférence 1 ou 2, tout particulièrement 1
et la double liaison peut aussi être substituée par un moins un résidu R
dans lequel un composé de formule (I) 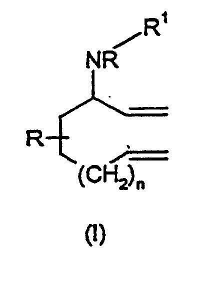 dans laquelle
R, R¹ et n ont la signification précitée
est soumis à une réaction de métathèse en présence d'un catalyseur de métaux nobles.

2. Procédé selon la revendication 1, **caractérisé en ce qu'**un complexe de ruthénium est utilisé comme catalyseur de métaux nobles.

3. Procédé selon l'une des revendications 1 et 2, **caractérisé en ce que** le complexe de métaux nobles est le dichlorure de bis(tricyclohexylphosphine)benzylidèneruthénium(IV).

4. Procédé selon l'une des revendications 1 à 3, **caractérisé en ce que** R dans les formules (I) et (II) désigne un hydrogène, un radical,éventuellement à noyaux condensés, aryle, alkyle, -CN, -COOR¹ ou un halogène.

5. Procédé selon l'une des revendications 1 à 4, **caractérisé en ce que** n a la signification 1 ou 2 dans les formules (I) et (II).

6. Procédé selon l'une des revendications 1 à 4, **caractérisé en ce qu** n a la signification 1 dans les formules (I) et (II).

7. Procédé selon l'une des revendications 1 à 6, **caractérisé en ce que** R¹ et R désignent l'hydrogène et n a la valeur 1 dans les formules (I) et (II).
